# EUROPEAN PATENT APPLICATION

(11) **EP 3 087 899 A1**
(43) Date of publication of application: **02.11.2016**
(21) Application number: 14875276.9
(22) Date of filing: 15.12.2014
(51) Int. Cl.: A61B 1/00, H02J 1/00, H04B 7/06

(54) **WIRELESS TRANSMITTER AND BIOLOGICAL INFORMATION-ACQUIRING SYSTEM**

(30) Priority: 27.12.2013 JP 2013271968
(71) Applicant: Olympus Corporation, Tokyo 192-8507 (JP)
(72) Inventor: SUGIYAMA Yuta, Hachioji-shi, Tokyo 192-8507 (JP); MATSUI Akira, Hachioji-shi, Tokyo 192-8507 (JP)
(74) Representative: Gunzelmann, Rainer
(86) International application number: PCT/JP2014/083123
(87) International publication number: WO 2015/098593

(57) **Abstract**

A wireless transmitter is arranged outside of a living body and configured to be able to perform wireless transmission utilizing magnetic coupling for an in-vivo observation apparatus inside of the living body, the wireless transmitter including: a magnetic field generation section including a plurality of plane coils inside, the plurality of plane coils being arranged such that each of coil surfaces is positioned on a same plane or arranged in a layer such that the coil surfaces are parallel to each other; a power source section configured to supply currents to be applied to the plurality of plane coils; a phase switch section configured to perform operation for supplying the currents supplied from the power source section to the plurality of plane coils while switching phases of the currents; and a control section configured to control the power source section and the phase switch section to sequentially and repeatedly switch states of the currents flowing through the plurality of plane coils at each predetermined period.

## Description

### Technical Field

The present invention relates to a wireless transmitter and a biological information acquisition system, and particularly, to a wireless transmitter and a biological information acquisition system that perform wireless transmission utilizing magnetic coupling.

### Background Art

In a medical field, a system with a configuration for performing wireless communication or wireless power feeding utilizing magnetic coupling between a transmission antenna (primary coil) provided on an apparatus arranged outside of a living body and a reception antenna (secondary coil) provided on another apparatus arranged in the living body has been conventionally proposed, for example.

More specifically, for example, Japanese Patent Application Laid-Open Publication No. 2010-125286 discloses a configuration of a living body observation system, in which magnetic coupling between a primary coil provided on a magnetic field generation section arranged outside of a living body and a secondary coil provided on a capsule endoscope arranged in the living body is utilized to switch on or off a power supply state of the capsule endoscope.

Furthermore, for example, Japanese Patent Application Laid-Open Publication No. 2008-283791 discloses a configuration of a wireless power feeding system for wireless power feeding using a magnetic field, in which a power transmission antenna that can cause a power reception antenna provided in a capsule endoscope to receive largest power is selected from a plurality of power transmission antennas arranged around a subject based on a detection result of a position and a direction of the capsule endoscope in a body of the subject.

However, according to the configuration disclosed in Japanese Patent Application Laid-Open Publication No. 2010-125286, the position and/or the posture of the capsule endoscope arranged in the living body can be changed in various ways, and there is a problem that the power supply state of the capsule endoscope cannot be switched to a desired power supply state in a situation in which the magnetic coupling between the primary coil and the secondary coil is extremely weak, for example.

When the configuration regarding the power transmission antenna of Japanese Patent Application Laid-Open Publication No. 2008-283791 is applied to the magnetic field generation section of Japanese Patent Application Laid-Open Publication No. 2010-125286 to solve the problem for example, there is a problem that the configuration of the system including the magnetic field generation section becomes complicated.

The present invention has been made in view of the circumstances, and an object of the present invention is to provide a wireless transmitter and a biological information acquisition system that can simply and surely perform wireless transmission utilizing magnetic coupling.

### Disclosure of Invention

### Means for Solving the Problem

An aspect of the present invention provides a wireless transmitter arranged outside of a living body and configured to be able to perform wireless transmission utilizing magnetic coupling for an in-vivo observation apparatus inside of the living body, the wireless transmitter including: a magnetic field generation section including a plurality of plane coils inside, the plurality of plane coils being arranged such that each of coil surfaces is positioned on a same plane or arranged in a layer such that the coil surfaces are parallel to each other; a power source section configured to supply the current to be applied to the plurality of plane coils; a phase switch section configured to perform operation for supplying the current supplied from the power source section to the plurality of plane coils while switching phases of the currents; and a control section configured to control the power source section and the phase switch section to sequentially and repeatedly switch states of the current flowing through the plurality of plane coils at each predetermined period.

An aspect of the present invention provides a biological information acquisition system including a wireless transmitter and an in-vivo observation apparatus and configured to be able to perform wireless transmission utilizing magnetic coupling between the wireless transmitter arranged outside of a living body and the in-vivo observation apparatus arranged inside of the living body, wherein the wireless transmitter including: a magnetic field generation section including a plurality of plane coils inside, the plurality of plane coils being arranged such that each of coil surfaces is positioned on a same plane or arranged in a layer such that the coil surfaces are parallel to each other; a power source section configured to supply the current to be applied to the plurality of plane coils; a phase switch section configured to perform operation for supplying the currents supplied from the power source section to the plurality of plane coils while switching phases of the currents; and a control section configured to control the power source section and the phase switch section to sequentially and repeatedly switch states of the currents flowing through the plurality of plane coils at each predetermined period.

### Brief Description of the Drawings

Fig. 1 is a diagram showing an outline of a biological information acquisition system according to an embodiment;
Fig. 2 is a diagram showing an example of an external configuration of a wireless transmitter according to the embodiment;
Fig. 3 is a diagram showing an example of an internal configuration of the wireless transmitter according to the embodiment;
Fig. 4 is a circuit diagram for describing an electrical connection relationship in the internal configuration of the wireless transmitter according to the embodiment;
Fig. 5 is a diagram showing an example of an internal configuration of a capsule endoscope according to the embodiment;
Fig. 6 is a diagram for describing an example of the external configuration of the wireless transmitter according to the embodiment, the example different from Fig. 2;
Fig. 7 is a diagram for describing an example of the internal configuration of the wireless transmitter according to the embodiment, the example different from Fig. 3;
Fig. 8 is an enlarged view of part of Fig. 7;
Fig. 9 is a diagram for describing an example of the internal configuration of the wireless transmitter according to the embodiment, the example different from Figs. 3 and 7;
Fig. 10 is a diagram for describing an arrangement state of a sheet member in Fig. 9;
Fig. 11 is a diagram showing an example of configuration of a transmission coil group that can be utilized in the wireless transmitter according to the embodiment, the example different from Fig. 3;
Fig. 12 is a diagram showing an example of configuration of a first transmission coil included in the transmission coil group that can be utilized in the wireless transmitter according to the embodiment;
Fig. 13 is a diagram showing an example of configuration of a second transmission coil included in the transmission coil group that can be utilized in the wireless transmitter according to the embodiment;
Fig. 14 is a diagram showing an example of configuration of a third transmission coil included in the transmission coil group that can be utilized in the wireless transmitter according to the embodiment; and
Fig. 15 is a diagram for describing a correspondence between a burst magnetic field generated from the wireless transmitter according to the embodiment and a pulse signal generated inside of the capsule endoscope.

### Best Mode for Carrying Out the Invention

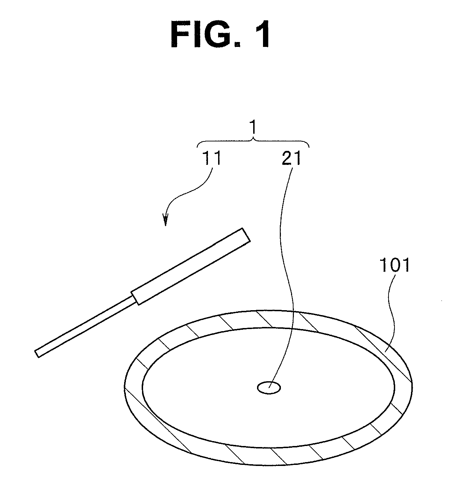

Hereinafter, an embodiment of the present invention will be described with reference to the drawings. Figs. 1 to 15 relate to the embodiment of the present invention.

As shown in Fig. 1, a biological information acquisition system 1 includes: a wireless transmitter 11 that generates a magnetic field outside of a patient 101 that is a living body; and a capsule endoscope 21 that can be arranged inside of the patient 101 and that switches on or off a power supply state according to the magnetic field generated from the wireless transmitter 11. Fig. 1 is a diagram showing an outline of the biological information acquisition system according to the embodiment.

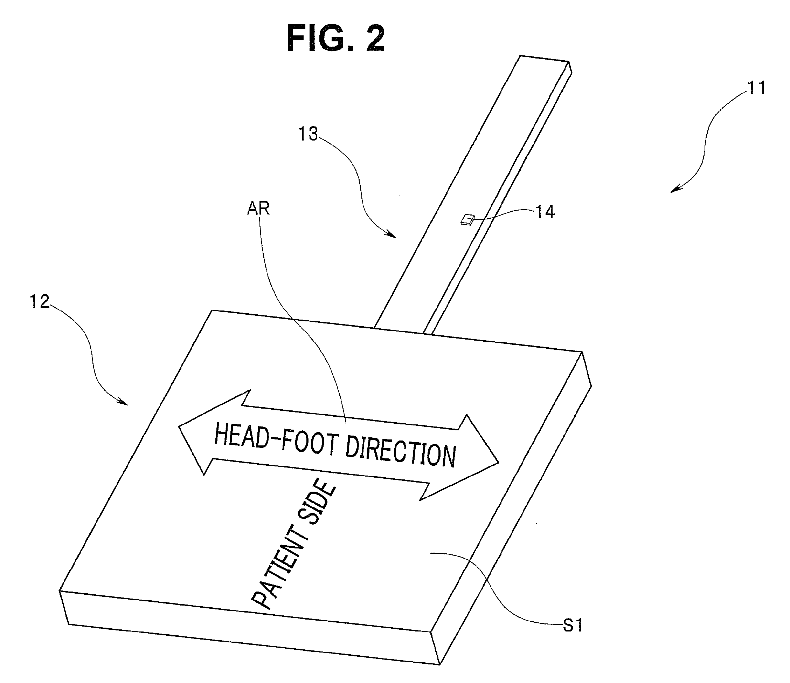

As shown in Fig. 2, the wireless transmitter 11 includes a magnetic field generation section 12 and a grasping section 13. Fig. 2 is a diagram showing an example of an external configuration of the wireless transmitter according to the embodiment.

An exterior of the magnetic field generation section 12 is formed by a resin or the like and is formed as a box-shaped hollow member in a substantially rectangular shape in plan view. A predetermined character string, such as a character string "patient side" of Fig. 2, is inscribed on a surface S 1 that is one of surfaces perpendicular to a thickness direction in the exterior of the magnetic field generation section 12, the character string allowing visual presentation for prompting use in a state that the surface S 1 opposes a body surface of the patient 101. Furthermore, a predetermined mark, such as a two-way arrow AR including a character string "head-foot direction" of Fig. 2, is inscribed on the surface S1, the mark allowing visual presentation for prompting use in a state in which a longitudinal direction of the patient 101 and an alignment direction of transmission coils 12A and 12B described later are parallel.

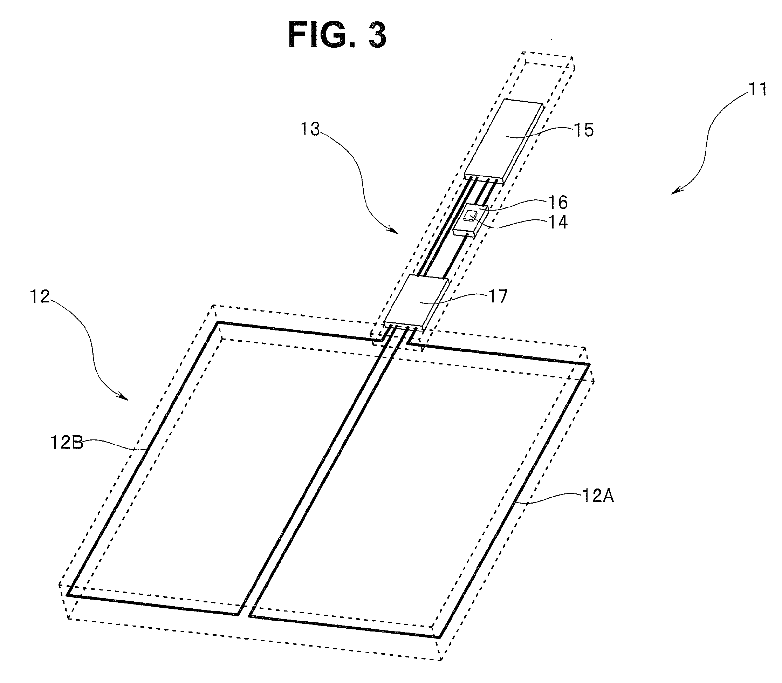

On the other hand, the transmission coils 12A and 12B are provided inside of the magnetic field generation section 12 as shown in Fig. 3. Fig. 3 is a diagram showing an example of an internal configuration of the wireless transmitter according to the embodiment.

The transmission coils 12A and 12B are formed as, for example, plane coils in a loop shape (coils in a shape with a thickness smaller than a length in a radial direction). The transmission coils 12A and 12B are arranged side by side in a direction indicated by the two-way arrow AR. That is, the two-way arrow AR inscribed on the surface S 1 of the magnetic field generation section 12 indicates an alignment direction of the transmission coils 12A and 12B provided inside of the magnetic field generation section 12. Furthermore, the transmission coils 12A and 12B are arranged such that each of coil surfaces is positioned on a same plane inside of the magnetic field generation section 12.

The grasping section 13 has a shape that allows a user, such as a surgeon, to grasp and is integrally formed with the magnetic field generation section 12.

An exterior of the grasping section 13 is formed by a resin or the like and is formed as a cylindrical hollow member in a substantially rectangular shape in plane view. An operation switch 14 pressed by the user, such as a surgeon, is provided on a surface on a same side as the surface S 1 on an exterior surface of the grasping section 13.

On the other hand, a power source section 15, a control section 16, and a phase switch section 17 are provided inside of the grasping section 13 as shown in Fig. 3.

The power source section 15 includes a battery, a waveform generator, and the like and is configured to be able to supply power required for driving the control section 16. The power source section 15 is configured to be able to supply an alternating current to the transmission coils 12A and 12B through the phase switch section 17. An amplitude of the alternating current is controlled by the control section 16.

The control section 16 includes a CPU, a memory, and the like and is configured to perform control for switching, at each predetermined period, the amplitude of the alternating current supplied from the power source section 15 to the phase switch section 17 and a phase of an alternating current supplied from the phase switch section 17 to the transmission coils 12A and 12B while the operation switch 14 is pressed.

More specifically, the control section 16 is configured to control the power source section 15 and the phase switch section 17 to perform operation of sequentially and repeatedly switching, at each predetermined period Tc, an in-phase mode in which alternating currents with an amplitude SL1 flow at a same phase (in a same direction) in the transmission coils 12A and 12B and a reverse-phase mode in which alternating currents with an amplitude SL2 (≥SL1) flow at phases opposite to each other (at directions opposite to each other) in the transmission coils 12A and 12B, while the operation switch 14 is pressed.

Note that according to the present embodiment, the amplitude SL2 is set to, for example, a value of about one to two times the amplitude SL1. According to the present embodiment, the predetermined period Tc is set to, for example, a value of about 1 to 100 msec.

The phase switch section 17 is configured to perform operation for supplying the alternating current supplied from the power source section 15 to each transmission coil (transmission coils 12A and 12B) while switching the phases according the control by the control section 16.

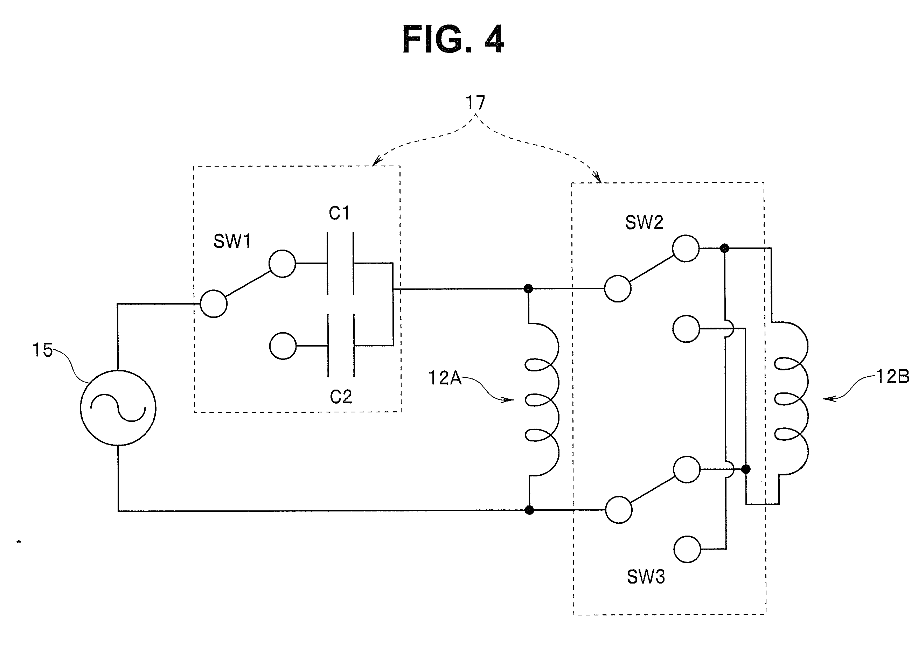

More specifically, the phase switch section 17 includes a resonant capacitor C1 and a resonant capacitor C2 connected parallel to each other, a switch SW1, a switch SW2, and a switch SW3 as shown for example in Fig. 4. On the other hand, the transmission coil 12B is connected parallel to the transmission coil 12A through the switches SW2 and SW3 as shown in Fig. 4. Fig. 4 is a circuit diagram for describing an electrical connection relationship in an internal configuration of the wireless transmitter according to the embodiment.

The switch SW1 is configured to turn on a contact point P1 during the in-phase mode according to control by the control section 16 to apply the alternating current supplied from the power source section 15 to the resonant capacitor C1. The switch SW1 is configured to turn on a contact point P2 during the reverse-phase mode according to control by the control section 16 to apply the alternating current supplied from the power source section 15 to the resonant capacitor C2.

The switches SW2 and SW3 are configured to turn on contact points P3 and P5 during the in-phase mode according to control by the control section 16 to apply the alternating currents with the same phase (in the same direction) to the transmission coils 12A and 12B. The switches SW2 and SW 3 are configured to turn on contact points P4 and P6 during the reverse-phase mode according to control by the control section 16 to apply the alternating currents with the phases opposite to each other (directions opposite to each other) to the transmission coils 12A and 12B.

The resonant capacitor C 1 has an electrostatic capacity that resonates with the transmission coils 12A and 12B at a predetermined resonant frequency RF (for example, 13.56 MHz) during the in-phase mode.

The resonant capacitor C2 has an electrostatic capacity that resonates with the transmission coils 12A and 12B at the predetermined resonant frequency RF (for example, 13.56 MHz) during the reverse-phase mode.

That is, according to the configuration, the alternating current with the amplitude SL1 passing through the resonant capacitor C1 flows to the transmission coils 12A and 12B during the in-phase mode, and magnetic fields with the frequency coinciding with the predetermined resonant frequency RF and in the same direction are generated from the transmission coils 12A and 12B. Furthermore, according to the configuration, the alternating current with the amplitude L2 passing through the resonant capacitor C2 flows to the transmission coils 12A and 12B during the reverse-phase mode, and magnetic fields with the frequency coinciding with the predetermined resonant frequency RF, in the directions opposite to each other, and with magnetic field strength equal to or greater than during the in-phase mode are generated from the transmission coils 12A and 12B.

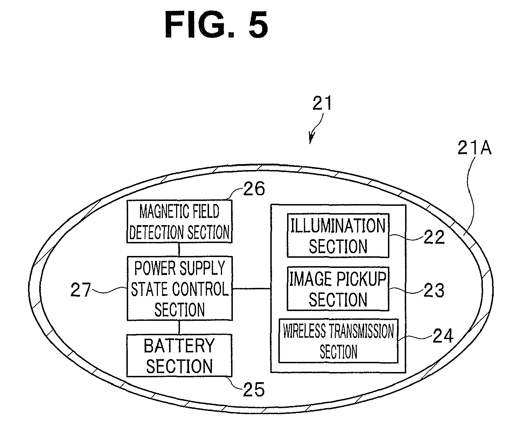

The capsule endoscope 21 includes a capsule housing 21A that can be arranged inside of the patient 101. As shown for example in Fig. 5, the capsule endoscope 21 includes, an illumination section 22, an image pickup section 23, a wireless transmission section 24, the illumination section 22, a battery section 25, a magnetic field detection section 26, and a power supply state control section 27, inside of the housing 21 A. Fig. 5 is a diagram showing an example of an internal configuration of the capsule endoscope according to the embodiment.

The illumination section 22 includes, for example, an LED and is configured to emit illuminating light for illuminating an object in a living body.

The image pickup section 23 includes, for example, a CCD and is configured to pick up and acquire an image of the object.

The wireless transmission section 24 includes, for example, a modulation circuit and is configured to wirelessly transmit the image acquired by the image pickup section 23 to the outside.

The battery section 25 includes, for example, cells and is configured to be able to supply power required for driving the illumination section 22, the image pickup section 23, and the wireless transmission section 24.

The magnetic field detection section 26 includes, for example, an LC resonant circuit that resonates at the predetermined resonant frequency RF and is configured to detect the magnetic field generated from the wireless transmitter 11 to output an electrical signal.

The power supply state control section 27 includes, for example, a CPU and is configured to perform operation for switching implementation and stop of the power supply by the battery section 25 when the electrical signal is continuously outputted from the magnetic field detection section 26 for more than a predetermined time period Ta.

That is, according to the configuration, the power supply state of the capsule endoscope 21 becomes an on-state when the power supply by the battery section 25 is implemented, and on the other hand, the power supply state of the capsule endoscope 21 becomes an off-state when the power supply by the battery section 25 is stopped. According to the configuration, before at least the predetermined time period Ta from the shift of the power supply state of the capsule endoscope 21 to the power supply state of one of the on-state and the off-state, the power supply state does not shift to the other power supply state.

Next, action of the biological information acquisition system 1 according to the present embodiment will be described.

In the state that the capsule endoscope 21 is arranged inside of the patient 101, the user moves the wireless transmitter 11 while grasping the grasping section 13 to thereby arrange the magnetic field generation section 12 in an arrangement state in which the surface S1 faces a body surface of the patient 101, and the two-way arrow AR inscribed on the surface S1 and the head-foot direction of the patient 101 coincide. After arranging the magnetic field generation section 12, the user presses the operation switch 14 while grasping the grasping section 13 to move the magnetic field generation section 12 in a circumferential direction of the patient 101.

On the other hand, the control section 16 controls the power source section 15 and the phase switch section 17 to perform the operation of sequentially and repeatedly switching the in-phase mode and the reverse-phase mode at each predetermined period Tc, when the operation switch 14 is pressed. According to the control by the control section 16, the transmission coils 12A and 12B sequentially and repeatedly (alternately) generate the magnetic fields in the same direction and the magnetic fields in the directions opposite to each other.

Here, a composite magnetic field during the reverse-phase mode includes more magnetic field components parallel to the coil surfaces of the transmission coils 12A and 12B, compared to a composite magnetic field during the in-phase mode. According to the present embodiment, magnetic fields with magnetic field strength equal to or greater than during the in-phase mode are generated from the transmission coils 12A and 12B during the reverse-phase mode. Therefore, according to the present embodiment, the power supply state of the capsule endoscope 21 can be surely switched even if the direction of the capsule endoscope 21 is changed to an arbitrary direction inside of the patient 101 due to a fast switch of the in-phase mode and the reverse-phase mode during a period in which the operation switch 14 is pressed.

According to the present embodiment, the power supply state of the capsule endoscope 21 arranged inside of the patient 101 can be switched without a complicated configuration of fixing and arranging coils that generate magnetic fields at predetermined positions around the patient 101, for example.

Therefore, according to the present embodiment, wireless transmission utilizing magnetic coupling can be simply and surely performed.

Note that the present embodiment is not limited to the control during the reverse-phase mode for supplying the alternating currents with the amplitude equal to or greater than the amplitude during the in-phase mode, and control for supplying alternating voltages with an amplitude equal to or greater than the amplitude during the in-phase mode may be performed, for example.

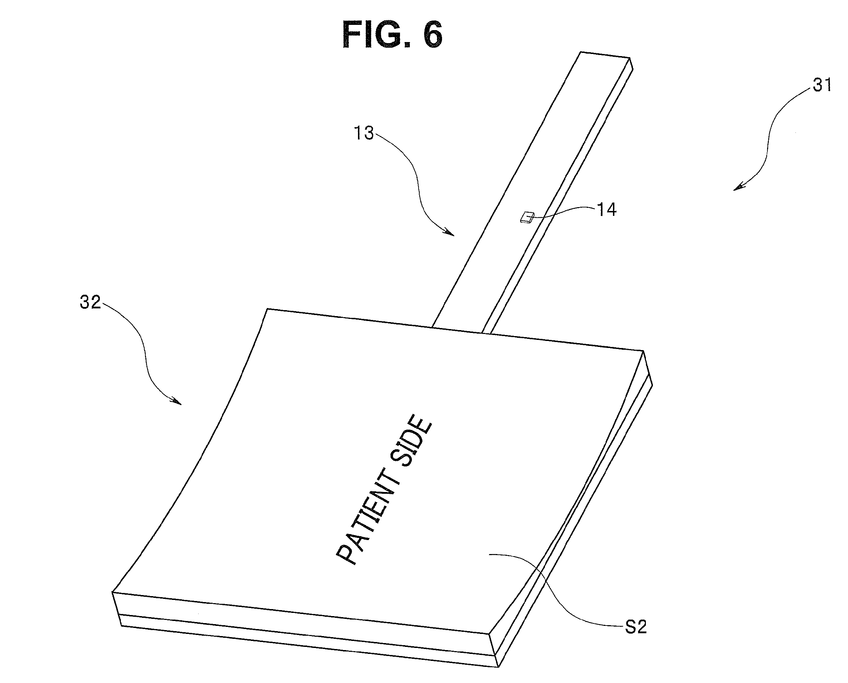

On the other hand, according to the present embodiment, a wireless transmitter 31 as shown for example in Fig. 6 may be used in place of the wireless transmitter 11 to form the biological information acquisition system 1. Fig. 6 is a diagram for describing an example of the external configuration of the wireless transmitter according to the embodiment, the example different from Fig. 2.

Note that detailed description of parts with same components as the components described above will be appropriately omitted for the simplification, and parts with configurations different from the configurations described above will be mainly described.

While the wireless transmitter 31 includes a magnetic field generation section 32 in place of the magnetic field generation section 12, the wireless transmitter 31 includes substantially the same components as those of the wireless transmitter 11 in each section other than the magnetic field generation section 32.

While the magnetic field generation section 32 includes a surface S2 on the exterior in place of the surface S1, the magnetic field generation section 32 includes substantially the same components as those of the magnetic field generation section 12 in each section other than the surface S2.

The surface S2 that is one of surfaces perpendicular to a thickness direction on the exterior of the magnetic field generation section 32 is formed as a bending surface bent in an arc shape relative to a direction orthogonal to the alignment direction of the transmission coils 12A and 12B provided inside of the magnetic field generation section 32. In other words, the surface S2 is formed in a shape that allows visual presentation for prompting the use in the state in which the longitudinal direction of the patient 101 and the alignment direction of the transmission coils 12A and 12B described later are parallel. A predetermined character string, such as a character string "patient side" of Fig. 6 is inscribed on the surface S2, allowing visual presentation for prompting the use in a state that the surface S2 opposes the body surface of the patient 101.

That is, according to the configuration, a curved surface of the surface S2 is opposite in the circumferential direction of the body surface of the patient 101, and the longitudinal direction (head-foot direction) of the patient 101 and the alignment direction of the transmission coil 12A and 12B can be parallel.

Therefore, according to the biological information acquisition system 1 using the wireless transmitter 31, same effects as in the biological information acquisition system 1 using the wireless transmitter 11 can be attained.

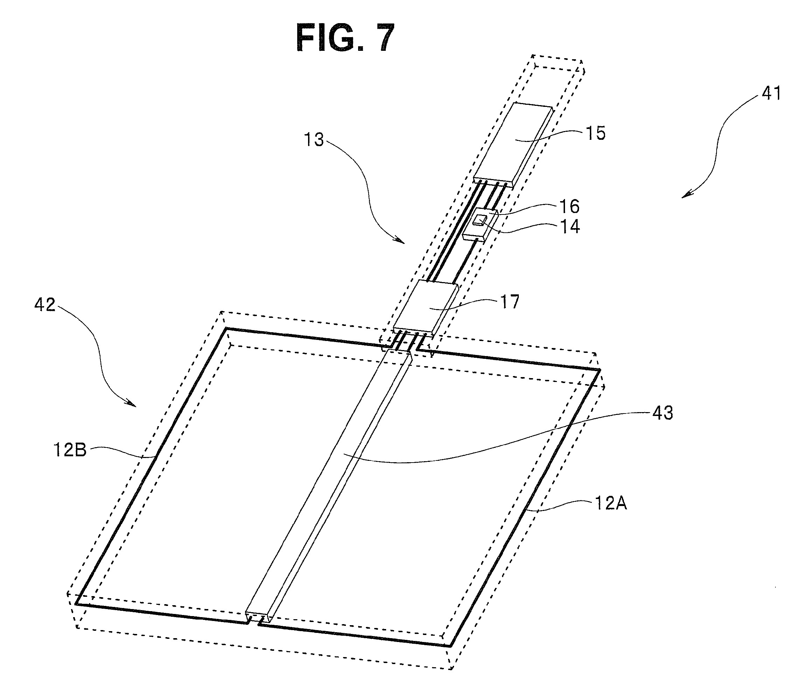

Furthermore, according to the present embodiment, a wireless transmitter 41 as shown for example in Fig. 7 may be used in place of the wireless transmitter 11 to form the biological information acquisition system 1. Fig. 7 is a diagram for describing an example of the internal configuration of the wireless transmitter according to the embodiment, the example different from Fig. 3.

While the wireless transmitter 41 includes a magnetic field generation section 42 in place of the magnetic field generation section 12, the wireless transmitter 41 includes substantially the same components as those of the wireless transmitter 11 in each section other than the magnetic field generation section 42.

While the magnetic field generation section 42 includes a conductive shield 43 inside, the magnetic field generation section 42 includes substantially the same components as those of the magnetic field generation section 12 in each section other than the conductive shield 43.

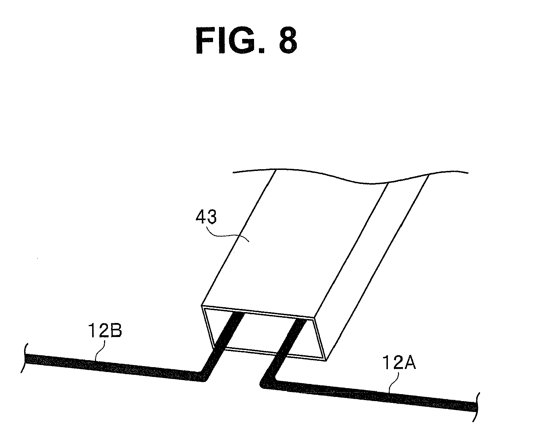

The conductive shield 43 is formed by highly conductive metal such as copper. The conductive shield 43 is formed as a tube body that can cover around a part where conductor wires of the transmission coils 12A and 12B are close to each other, as shown for example in Figs. 7 and 8. Fig. 8 is an enlarged view of part of Fig. 7.

Therefore, according to the biological information acquisition system 1 using the wireless transmitter 41, the same effects as in the biological information acquisition system 1 using the wireless transmitter 11 can be attained, and generation of unnecessary electric fields associated with the supply of the alternating currents to the transmission coils 12A and 12B can be prevented.

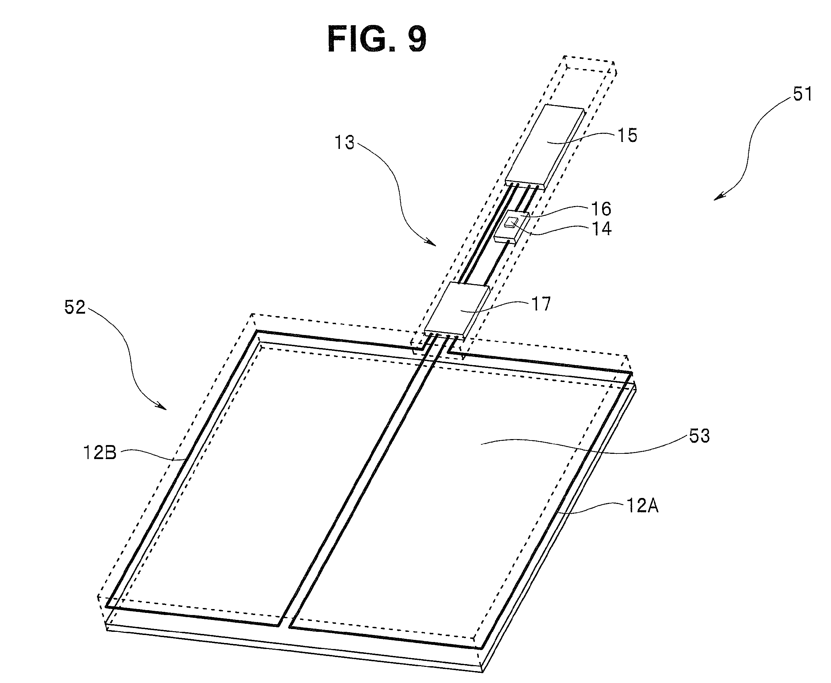

Furthermore, according to the present embodiment, a wireless transmitter 51 as shown for example in Fig. 9 may be used in place of the wireless transmitter 11 to form the biological information acquisition system 1. Fig. 9 is a diagram for describing an example of an internal configuration of the wireless transmitter according to the embodiment, the example different from Figs. 3 and 7.

While the wireless transmitter 51 includes a magnetic field generation section 52 in place of the magnetic field generation section 12, the wireless transmitter 51 includes substantially the same components as those of the wireless transmitter 11 in each section other than the magnetic field generation section 52.

While the magnetic field generation section 52 includes a sheet member 53 formed by a soft magnetic material inside, the magnetic field generation section 52 includes substantially the same components as those of the magnetic field generation section 12 in each section other than the sheet member 53.

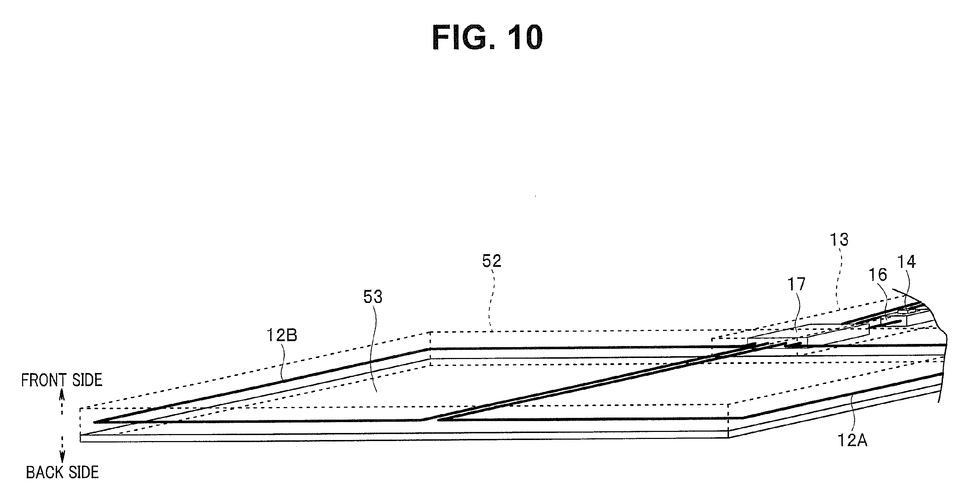

As shown for example in Figs. 9 and 10, assuming that a surface S1 (surface with character string "patient side") side of the magnetic field generation section 52 is a front side of the coil surface of each of the transmission coils (transmission coils 12A and 12B), the sheet member 53 has a shape covering an entire back side of the coil surface of each of the transmission coils (transmission coils 12A and 12B) inside of the magnetic field generation section 52. Fig. 10 is a diagram for describing an arrangement state of the sheet member in Fig. 9.

Therefore, according to the biological information acquisition system 1 using the wireless transmitter 51, the same effects as in the biological information acquisition system 1 using the wireless transmitter 11 can be attained, and generation of unnecessary electric fields associated with the supply of the alternating currents to the transmission coils 12A and 12B can be prevented.

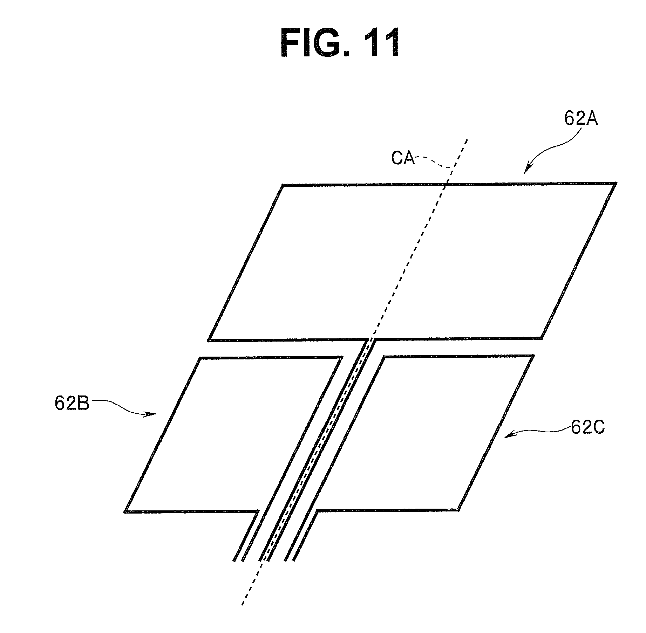

On the other hand, according to the present embodiment, transmission coils 62A, 62B, and 62C may be provided inside of the magnetic field generation section 12 in place of the transmission coils 12A and 12B as shown for example in Fig. 11, and control according to the transmission coils 62A, 62B, and 62C may be performed. Fig. 11 is a diagram showing an example of configuration of a transmission coil group that can be utilized in the wireless transmitter according to the embodiment, the example different from Fig. 3.

The transmission coils 62A, 62B, and 62C are arranged such that each of coil surfaces is positioned on a same plane inside of the magnetic field generation section 12. Although not shown, the transmission coils 62A, 62B, and 62C are connected parallel to each other. Although not shown, each of the transmission coils 62A, 62B, and 62C forms a resonant circuit that resonates at the predetermined resonant frequency RF when the transmission coils 62A, 62B, and 62C are connected to one or more resonant capacitors provided in the phase switch section 17.

The transmission coil 62A is formed as a plane coil in a loop shape. The transmission coil 62A is formed to have a coil surface with an area greater than (for example, substantially twice) the areas of the transmission coils 62B and 62C and is arranged at a position farther from the grasping section 13 than the transmission coils 62B and 62C.

Each of the transmission coils 62B and 62C is formed as a plane coil in a loop shape. The transmission coils 62B and 62C are formed to have coil surfaces with substantially a same area and are arranged at positions horizontally symmetrical about a center axis (equivalent to an axis CA of Fig. 11) in a longitudinal direction of the grasping section 13. That is, according to the configuration, the two-way arrow AR (not shown in Fig. 11) inscribed on the surface S 1 indicates a direction coinciding with an alignment direction of the transmission coils 62B and 62C.

On the other hand, when the operation switch 14 is pressed, the control section 16 sequentially and repeatedly switches three modes including a Z axis mode, an X axis mode, and a Y axis mode at each predetermined time period Td and controls the power source section 15 and the phase switch section 17 to supply an alternating current with an amplitude and a phase according to each mode to the transmission coils 62A, 62B, and 62C.

More specifically, the control section 16 controls the power source section 15 and the phase switch section 17 to apply alternating currents with an amplitude SL3 and with the same phase to the transmission coils 62A, 62B, and 62C in the Z axis mode. According to the control by the control section 16, magnetic fields in the same direction are generated from the transmission coils 62A, 62B, and 62C.

In the X axis mode, the control section 16 controls the power source section 15 and the phase switch section 17 to apply alternating currents with an amplitude SL4 (≥SL3) and with phases opposite to each other to the transmission coils 62B and 62C and not to apply a current to the transmission coil 62A (for example, control for opening a coil end of the transmission coil 62A). According to the control by the control section 16, magnetic fields in directions opposite to each other are generated from the transmission coils 62B and 62C.

In the Y axis mode, the control section 16 controls the power source section 15 and the phase switch section 17 to apply alternating currents with an amplitude SL5 (≥SL3) and with the same phase to the transmission coils 62B and 62C and to apply an alternating current with a phase opposite to the transmission coil 62B (and 62C) to the transmission coil 62A. According to the control by the control section 16, magnetic fields in the same direction are generated from the transmission coils 62B and 62C, and a magnetic field in a direction opposite to the direction of the magnetic fields generated from the transmission coils 62B and 62C is generated from the transmission coil 62A.

Note that according to the present embodiment, the amplitude SL4 is set to, for example, a value of about one to two times the amplitude SL3. According to the present embodiment, the amplitude SL5 is set to, for example, a value of about one to three times the amplitude SL3. According to the present embodiment, the predetermined time period Td is set to, for example, a value of about 10 msec.

That is, according to the configuration with the transmission coils 62A, 62B, and 62C, when the magnetic field generation section 12 is arranged in the arrangement state in which the surface S 1 opposes the body surface of the patient 101, and the two-way arrow AR inscribed on the surface S1 and the head-foot direction of the patient 101 coincide for example, the operation switch 14 can be pressed to switch the power supply state of the capsule endoscope 21 without operation of moving the magnetic field generation section 12 in the circumferential direction of the patient 101.

Therefore, even when the transmission coils 62A, 62B, and 62C are provided inside of the magnetic field generation section 12, the same effects as when the transmission coils 12A and 12B are provided inside of the magnetic field generation section 12 are attained.

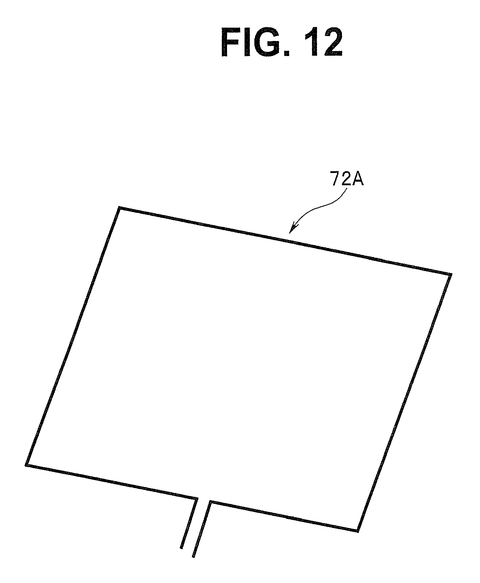

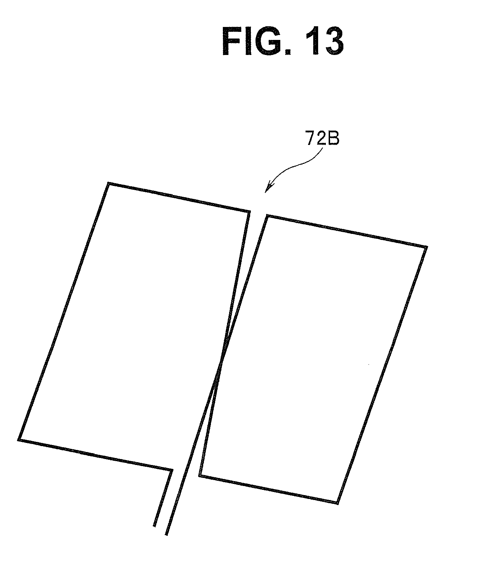

According to the present embodiment, for example, a transmission coil 72A as shown in Fig. 12 and a transmission coil 72B as shown in Fig. 13 may be provided inside of the magnetic field generation section 12 in place of the transmission coils 12A and 12B, and control according to the transmission coils 72A and 72B may be performed. Fig. 12 is a diagram showing an example of configuration of a first transmission coil included in a transmission coil group that can be utilized in the wireless transmitter according to the embodiment. Fig. 13 is a diagram showing an example of configuration of a second transmission coil included in the transmission coil group that can be utilized in the wireless transmitter according to the embodiment.

Although not shown, the transmission coils 72A and 72B are arranged in a layer such that coil surfaces are parallel to each other inside of the magnetic field generation section 12. Although not shown, the transmission coils 72A and 72B are connected parallel to each other. Although not shown, each of the transmission coils 72A and 72B forms a resonant circuit that resonates at the predetermined resonant frequency RF when the transmission coils 72A and 72B are connected to one or more resonant capacitors provided in the phase switch section 17.

The transmission coil 72A is formed as a plane coil in a loop shape.

The transmission coil 72B is formed as a plane coil in an 8-shape in which a conductive path intersects at a coil center portion as viewed in the direction indicated by the two-way arrow AR (not shown in Figs. 12 and 13) inscribed on the surface S1.

Note that according to the configuration including the transmission coils 72A and 72B, the sheet member 53 may be provided to cover the entire back side of the coil surface of the transmission coil provided closest to the back, inside of the magnetic field generation section 12, for example.

On the other hand, when the operation switch 14 is pressed, the control section 16 controls the power source section 15 and the phase switch section 17 to perform operation of sequentially and repeatedly switching a mode of applying an alternating current with the amplitude SL1 only to the transmission coil 72A and a mode of applying an alternating current with the amplitude SL2 only to the transmission coil 72B at each predetermined period Te. When the alternating current flows through one of the transmission coils 72A and 72B, the control section 16 performs one of control for opening the coil end of the other transmission coil, control for connecting a resistance component to the coil end of the other transmission coil, and control for connecting a reactance component to the coil end of the other transmission coil, for example.

That is, according to the configuration with the transmission coils 72A and 72B, a state in which the magnetic field in the direction perpendicular to the coil surface at the coil center portion is generated from the transmission coil 72A and a state in which the magnetic field in the direction parallel to the coil surface at the coil center portion is generated from the transmission coil 72B are sequentially repeated when the operation switch 14 is pressed.

Therefore, even when the transmission coils 72A and 72B are provided inside of the magnetic field generation section 12, the same effects as when the transmission coils 12A and 12B are provided inside of the magnetic field generation section 12 are attained.

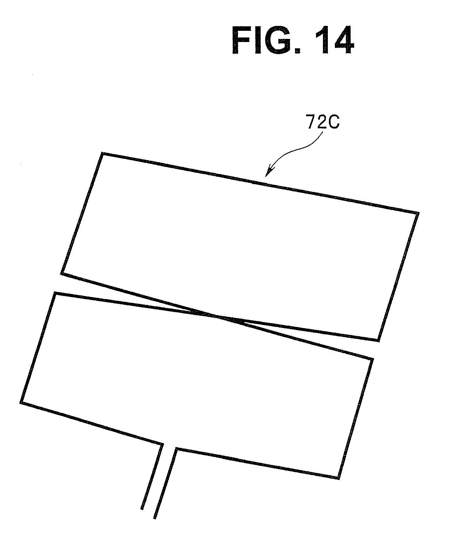

According to the present embodiment, for example, the transmission coil 72A as shown in Fig. 12, the transmission coil 72B as shown in Fig. 13, and a transmission coil 72C as shown in Fig. 14 may be provided inside of the magnetic field generation section 12 (in place of the transmission coils 12A and 12B), and control according to the transmission coils 72A to 72C may be performed. Fig. 14 is a diagram showing an example of configuration of a third transmission coil included in the transmission coil group that can be utilized in the wireless transmitter according to the embodiment.

Although not shown, the transmission coils 72A to 72C are arranged in a layer such that the coil surfaces are parallel to each other inside of the magnetic field generation section 12. Although not shown, the transmission coils 72A to 72C are connected parallel to each other. Although not shown, each of the transmission coils 72A to 72C forms a resonant circuit that resonates at the predetermined resonant frequency RF when the transmission coils 72A to 72C are connected to one or more resonant capacitors provided in the phase switch section 17.

The transmission coil 72C is formed as a plane coil in an 8-shape in which a conductive path intersects at a coil center portion as viewed in the direction orthogonal to the direction indicated by the two-way arrow AR (not shown in Fig. 14) inscribed on the surface S1.

Note that according to the configuration including the transmission coils 72A to 72C, the sheet member 53 may be provided to cover the entire back side of the coil surface of the transmission coil provided closest to the back, inside of the magnetic field generation section 12, for example.

On the other hand, when the operation switch 14 is pressed, the control section 16 controls the power source section 15 and the phase switch section 17 to perform operation of sequentially and repeatedly switching the mode of applying the alternating current with the amplitude SL1 only to the transmission coil 72A, the mode of applying the alternating current with the amplitude SL2 only to the transmission coil 72B, and a mode of applying an alternating current with the amplitude SL2 only to the transmission coil 72C at each predetermined period Tf.

When the alternating current flows through one of the transmission coils 72A to 72C, the control section 16 performs one of control for opening each of the coil ends of the other two transmission coils, control for connecting a resistance component to each of the coil ends of the other two transmission coils, and control for connecting a reactance component to each of the coil ends of the other two transmission coils, for example.

That is, according to the configuration with the transmission coils 72A to 72C, the state in which the magnetic field in the direction perpendicular to the coil surface at the coil center portion is generated from the transmission coil 72A, the state in which the magnetic field in the direction parallel to the coil surface at the coil center portion is generated from the transmission coil 72B, and a state in which a magnetic field in a direction orthogonal to each of the directions of the magnetic fields generated from the transmission coils 72A and 72B at the coil center portion is generated from the transmission coil 72C are sequentially repeated when the operation switch 14 is pressed.

That is, according to the configuration with the transmission coils 72A to 72C, when the magnetic field generation section 12 is arranged in the arrangement state in which the surface S1 opposes the body surface of the patient 101, and the two-way arrow AR inscribed on the surface S1 and the head-foot direction of the patient 101 coincide for example, the power supply state of the capsule endoscope 21 can be switched by pressing the operation switch 14, without operation for moving the magnetic field generation section 12 in the circumferential direction of the patient 101.

Therefore, even when the transmission coils 72A to 72C are provided inside of the magnetic field generation section 12, the same effects as when the transmission coils 12A and 12B are provided inside of the magnetic field generation section 12 are attained.

Note that the present embodiment may be appropriately modified to switch an emission state of the illuminating light from the capsule endoscope 21 according to the magnetic field generated from the wireless transmitter 11, for example. The present embodiment may be appropriately modified to switch a frame rate regarding the acquisition of the image by the capsule endoscope 21 according to the magnetic field generated from the wireless transmitter 11, for example.

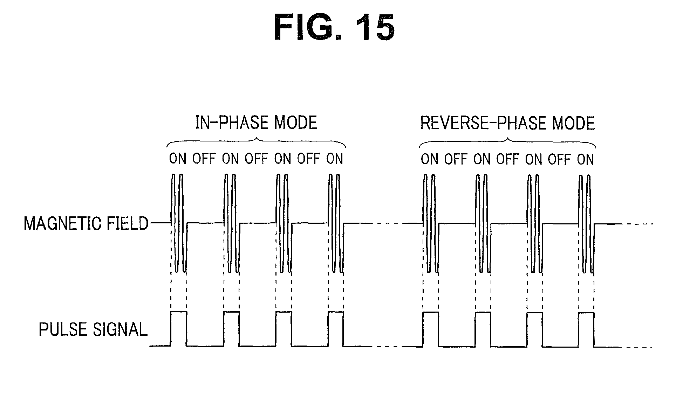

The configuration of the wireless transmitter 11, 31, 41, or 51 may be appropriately modified to perform control of alternately switching on or off electrical connection between each coil and the phase switch section 17 in each mode of the in-phase mode and the reverse-phase mode, and a burst (intermittent) magnetic field according to the control may be generated as shown for example in Fig. 15. According to the configuration, the magnetic field detection section 26 may output, for example, a pulse signal with the number of pulses coinciding with the number of times of generation of the burst (intermittent) magnetic field in the capsule endoscope 21, and the power supply state, the emission state of the illuminating light, and (or) the frame rate regarding the acquisition of the image may be controlled based on the number of pulses. Fig. 15 is a diagram for describing a correspondence between the burst magnetic field generated from the wireless transmitter according to the present embodiment and the pulse signal generated inside of the capsule endoscope.

The present embodiment is not limited to the case in which the number of turns of the transmission coil is one, and for example, the present embodiment can be substantially similarly applied when the number of turns of the transmission coil is two or more, such as in a spiral coil and a helical coil.

The present invention is not limited to each of the embodiments, and it is obvious that various changes and applications are possible without departing from the scope of the invention.

The present application is filed on the basis of claiming the benefit of priority from Japanese Patent Application No. 2013-271968, filed December 27, 2013 in Japan, and the disclosed contents are incorporated into the present specification, the claims, and the drawings by reference.

## Claims

1. A wireless transmitter arranged outside of a living body and configured to be able to perform wireless transmission utilizing magnetic coupling for an in-vivo observation apparatus inside of the living body, the wireless transmitter comprising:
a magnetic field generation section including a plurality of plane coils inside, the plurality of plane coils being arranged such that each of coil surfaces is positioned on a same plane or arranged in a layer such that the coil surfaces are parallel to each other;
a power source section configured to supply the current to be applied to the plurality of plane coils;
a phase switch section configured to perform operation for supplying the current supplied from the power source section to the plurality of plane coils while switching phases of the currents; and
a control section configured to control the power source section and the phase switch section to sequentially and repeatedly switch states of the current flowing through the plurality of plane coils at each predetermined period.

2. The wireless transmitter according to claim 1, wherein
the magnetic field generation section includes two plane coils arranged such that each of coil surfaces is positioned on a same plane, and
the control section controls the power source section and the phase switch section to sequentially and repeatedly switch, at the each predetermined period, an in-phase state in which currents with a same phase flow through the two plane coils and a reverse-phase state in which currents with opposite phases flow through the two plane coils.

3. The wireless transmitter according to claim 2, wherein
during the reverse-phase state, the control section controls the power source section to supply currents with an amplitude equal to or greater than an amplitude during the in-phase state.

4. The wireless transmitter according to claim 2, wherein
a predetermined mark that allows visual presentation for prompting use in a state in which a longitudinal direction of the living body and an alignment direction of the two plane coils are parallel is inscribed on an exterior.

5. The wireless transmitter according to claim 2, wherein
at least part of the exterior of the magnetic field generation section has a shape that allows the visual presentation for prompting the use in the state in which the longitudinal direction of the living body and the alignment direction of the two plane coils are parallel.

6. The wireless transmitter according to claim 1, wherein
the magnetic field generation section comprises three plane coils arranged such that each of coil surfaces is arranged on the same plane, and
the control section controls the power source section and the phase switch section for sequentially and repeatedly switching, at the each predetermined period, a state in which current with a same phase flow through the three plane coils, a state in which currents with opposite phases flow through predetermined two plane coils among the three plane coils and a current does not flow through another one plane coil, and a state in which currents with a same phase flow through the predetermined two plane coils and a current with an opposite phase flows through the other one plane coil.

7. The wireless transmitter according to claim 1, wherein
the magnetic field generation section comprises: a first plane coil that generates a magnetic field in which a direction of the magnetic field at a coil center portion is perpendicular to a coil surface; and a second plane coil that generates a magnetic field in which a direction of the magnetic field at a coil center portion is parallel to a coil surface, wherein the first plane coil and the second plane coil are arranged in a layer such that the coil surfaces are parallel to each other, and
the control section controls the power source section and the phase switch section to sequentially and repeatedly switch, at the each predetermined period, a state in which a current flows through only the first plane coil and a state in which a current flows through only the second plane coil.

8. The wireless transmitter according to claim 1, wherein
the magnetic field generation section comprises: a first plane coil that generates a magnetic field in which a direction of the magnetic field at a coil center portion is perpendicular to a coil surface; a second plane coil that generates a magnetic field in which a direction of the magnetic field at a coil center portion is parallel to a coil surface; and a third plane coil that generates a magnetic field in which a direction of the magnetic field at a coil center portion is orthogonal to each of the directions of the magnetic fields generated from the first plane coil and the second plane coil, wherein the first plane coil, the second plane coil, and the third plane coil are arranged in a layer such that the coil surfaces are parallel to each other, and
the control section controls the power source section and the phase switch section to sequentially and repeatedly switch, at the each predetermined period, a state in which a current flows through only the first plane coil, a state in which a current flows through only the second plane coil, and a state in which a current flows through only the third plane coil.

9. The wireless transmitter according to claim 1, wherein
the magnetic field generation section comprises a conductive shield inside, the conductive shield covering around a part where conductor wires consisted of the plurality of plane coils are close to each other.

10. The wireless transmitter according to claim 2, wherein
the magnetic field generation section is formed by a soft magnetic material and comprises a sheet member inside, wherein the sheet member shaped for covering an entire back side of each of the coil surfaces of the two plane coils.

11. A biological information acquisition system comprising a wireless transmitter and an in-vivo observation apparatus and configured to be able to perform wireless transmission utilizing magnetic coupling between the wireless transmitter arranged outside of a living body and the in-vivo observation apparatus arranged inside of the living body,
wherein he wireless transmitter comprising:
a magnetic field generation section including a plurality of plane coils inside, the plurality of plane coils being arranged such that each of coil surfaces is positioned on a same plane or arranged in a layer such that the coil surfaces are parallel to each other;
a power source section configured to supply the current to be applied to the plurality of plane coils;
a phase switch section configured to perform operation for supplying the currents supplied from the power source section to the plurality of plane coils while switching phases of the currents; and
a control section configured to control the power source section and the phase switch section to sequentially and repeatedly switch states of the currents flowing through the plurality of plane coils at each predetermined period.

## Amended claims

### Amended claims under Art. 19.1 PCT

**1.** (Amended) A wireless transmitter that is arranged outside of a living body and that switches state of power supply for an in-vivo observation apparatus arranged inside of the living body, the wireless transmitter comprising:
a magnetic field generation section comprising a plurality of plane coils inside, the plurality of plane coils generating a magnetic field by current, wherein each of coil surfaces are arranged on a same plane, or the coil surfaces are arranged parallel to each other in a layer;
a power source section that supplies the current to be applied to the plurality of plane coils;
a phase switch section that switches phases of the current supplied from the power source section; and
a control section that controls the phase switch section to switch, at each predetermined period, a state of the current flowing through the plurality of plane coils between an in-phase state in which currents with a same phase flow and a reverse-phase state in which currents with opposite phases flow to thereby alternately generate magnetic fields in a same direction and magnetic fields in directions opposite to each other in the plurality of plane coils, wherein
the control section controls the power source section to supply currents with an amplitude equal to or greater than an amplitude of the in-phase state during the reverse-phase state.

**2.** (Amended) The wireless transmitter according to claim 1, wherein
the magnetic field generation section includes two plane coils arranged such that each of coil surfaces is positioned on a same plane.

**3.** (Canceled)

**4.** The wireless transmitter according to claim 2, wherein
a predetermined mark that allows visual presentation for prompting use in a state in which a longitudinal direction of the living body and an alignment direction of the two plane coils are parallel is inscribed on an exterior.

**5.** The wireless transmitter according to claim 2, wherein
at least part of the exterior of the magnetic field generation section has a shape that allows the visual presentation for prompting the use in the state in which the longitudinal direction of the living body and the alignment direction of the two plane coils are parallel.

**6.** The wireless transmitter according to claim 1, wherein
the magnetic field generation section comprises three plane coils arranged such that each of coil surfaces is arranged on the same plane, and
the control section controls the power source section and the phase switch section for sequentially and repeatedly switching, at the each predetermined period, a state in which current with a same phase flow through the three plane coils, a state in which currents with opposite phases flow through predetermined two plane coils among the three plane coils and a current does not flow through another one plane coil, and a state in which currents with a same phase flow through the predetermined two plane coils and a current with an opposite phase flows through the other one plane coil.

**7.** The wireless transmitter according to claim 1, wherein
the magnetic field generation section comprises:
a first plane coil that generates a magnetic field in which a direction of the magnetic field at a coil center portion is perpendicular to a coil surface; and
a second plane coil that generates a magnetic field in which a direction of the magnetic field at a coil center portion is parallel to a coil surface,
wherein the first plane coil and the second plane coil are arranged in a layer such that the coil surfaces are parallel to each other, and
the control section controls the power source section and the phase switch section to sequentially and repeatedly switch, at the each predetermined period, a state in which a current flows through only the first plane coil and a state in which a current flows through only the second plane coil.

**8.** The wireless transmitter according to claim 1, wherein
the magnetic field generation section comprises:
a first plane coil that generates a magnetic field in which a direction of the magnetic field at a coil center portion is perpendicular to a coil surface;
a second plane coil that generates a magnetic field in which a direction of the magnetic field at a coil center portion is parallel to a coil surface; and
a third plane coil that generates a magnetic field in which a direction of the magnetic field at a coil center portion is orthogonal to each of the directions of the magnetic fields generated from the first plane coil and the second plane coil,
wherein the first plane coil, the second plane coil, and the third plane coil are arranged in a layer such that the coil surfaces are parallel to each other, and
the control section controls the power source section and the phase switch section to sequentially and repeatedly switch, at the each predetermined period, a state in which a current flows through only the first plane coil, a state in which a current flows through only the second plane coil, and a state in which a current flows through only the third plane coil.

**9.** The wireless transmitter according to claim 1, wherein
the magnetic field generation section comprises a conductive shield inside, the conductive shield covering around a part where conductor wires consisted of the plurality of plane coils are close to each other.

**10.** The wireless transmitter according to claim 2, wherein
the magnetic field generation section is formed by a soft magnetic material and comprises a sheet member inside,
wherein the sheet member shaped for covering an entire back side of each of the coil surfaces of the two plane coils.

**11.** (Amended) A biological information acquisition system comprising:
a wireless transmitter arranged outside of a living body; and
an in-vivo observation apparatus arranged inside of the living body,
wherein the wireless transmitter switching state of power supply for the in-vivo observation apparatus,
wherein the wireless transmitter comprising:
a magnetic field generation section comprising a plurality of plane coils inside, the plurality of plane coils generating a magnetic field by current,
wherein each of coil surfaces are arranged on a same plane, or the coil surfaces are arranged parallel to each other in a layer;
a power source section that supplies the current to be applied to the plurality of plane coils;
a phase switch section that switches phases of the currents supplied from the power source section; and
a control section that controls the phase switch section to switch, at each predetermined period, a state of the currents flowing through the plurality of plane coils between an in-phase state in which currents with a same phase flow and a reverse-phase state in which currents with opposite phases flow to thereby alternately generate magnetic fields in a same direction and magnetic fields in directions opposite to each other in the plurality of plane coils, wherein
the control section controls the power source section to supply currents with an amplitude equal to or greater than an amplitude of the in-phase state during the reverse-phase state.
